Europäisches Patentamt

European Patent Office

Office européen des brevets

(19)

(11) EP 0 597 374 B1

(12) **EUROPÄISCHE PATENTSCHRIFT**

(45) Veröffentlichungstag und Bekanntmachung des Hinweises auf die Patenterteilung:
**20.03.1996 Patentblatt 1996/12**

(51) Int Cl.$^6$: **G05D 11/13**, B01F 3/02, A61M 16/12

(21) Anmeldenummer: **93117809.9**

(22) Anmeldetag: **03.11.1993**

(54) **Verfahren und Vorrichtung zur Mischung von Gasen**

Method and device for mixing gases

Méthode et dispositif pour le mélange des gaz

(84) Benannte Vertragsstaaten:
**AT CH DE FR GB LI**

(30) Priorität: **11.11.1992 DE 4238011**

(43) Veröffentlichungstag der Anmeldung:
**18.05.1994 Patentblatt 1994/20**

(73) Patentinhaber: **Linde Aktiengesellschaft**
**D-65189 Wiesbaden (DE)**

(72) Erfinder:
- **Sadjina, Heinz, Dipl.-Ing.**
 **D-82049 Pullach (DE)**
- **Hildebrandt, Peter, Dr.-Ing.**
 **D-85402 Kranzberg (DE)**

(74) Vertreter: **Kasseckert, Rainer**
**Linde Aktiengesellschaft,**
**Zentrale Patentabteilung**
**D-82049 Höllriegelskreuth (DE)**

(56) Entgegenhaltungen:
EP-A- 0 343 542          FR-A- 2 532 858
FR-A- 2 548 549          GB-A- 2 026 729
GB-A- 2 053 512

- PATENT ABSTRACTS OF JAPAN vol. 10, no. 148 (C-350) (2205) 29. Mai 1986 & JP-A-61 008 127 (OVAL KIKI KOGYO K.K.) 14. Januar 1986
- PATENT ABSTRACTS OF JAPAN vol. 9, no. 116 (P-357) (1839) 21. Mai 1985 & JP-A-60 003 011 (TOSHIBA K.K.) 9. Januar 1985
- PATENT ABSTRACTS OF JAPAN vol. 9, no. 214 (P-384) (1937) 31. August 1985 & JP-A-60 073 712 (TOSHIBA K.K.) 25. April 1985

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Mischung von zwei oder mehr Gasen in geregelter Weise, wobei die Flüsse der unvermischten Gase geeignet im Hinblick auf das gewünschte Mischungsverhältnis eingestellt werden, sowie eine dementsprechende Regelvorrichtung.

Für die, wie eben beschriebene, Bereitstellung von Gasmischungen sind - gerade vor dem Hintergrund unterschiedlichster Gase und Einsatzbereiche - bereits ein Vielzahl Lösungen bekannt (siehe z.B. die DE-A 23 51 351 oder die DE-PS 25 53 165). Bei der Bereitstellung einer Gasmischung aus den getrennt vorhandenen Komponenten stellt zum einen die Mischgenauigkeit eine wesentliche Zielgröße dar, andererseits ist auch die Fähigkeit schwankenden Bedarfsmengen schnell und exakt folgen zu können wichtig. Bei vielen Gasmischsystemen oder Gasmischern treten jedoch trotz besonderer Berücksichtigung dieser Zielgrößen unbefriedigende Ergebnisse im praktischen Einsatz auf.

Ein beispielsweise aus FR-A-2 532 858 bekannter Lösungsvorschlag für verschiedenste Gasmischverfahren bzw. Gasmischer, der gerade diesen eben angesprochenen, beiden Zielanforderungen sehr gut nachkommt, besteht darin, einen Puffer- oder Speicherbehälter nach dem eigentlichen Gasmischer anzuordnen und so die von einem oder mehreren Verbrauchern gegebenenfalls plötzlich angeforderten Gasmengen auch in der Zeit der größten Mengenänderung im richtigen Mischungsverhältnis und ohne Druckabfall liefern zu können. Die Anwendung eines Pufferbehälters stellt jedoch einen erheblichen Zusatzaufwand dar und schlägt hinsichtlich der Baugröße einer Gasmischeinheit wesentlich zu Buche.

Die Aufgabenstellung der vorliegenden Erfindung besteht daher darin, ein Gasmischverfahren bzw.eine zugehörige Regelvorrichtung zu gestalten, das bzw. die die Bereitstellung einer Gasmischung mit großer Mischungsgenauigkeit auch bei variierenden Gasmengen leistet.

Diese Aufgabenstellung wird erfindungsgemäß dadurch gelöst, daß - unter Vorgabe der Festlegung, daß das Gas mit dem größten Mengenanteil in der Mischung das Mastergas darstellt und daß das(die) weitere(n) Gas (e) in der Mischung das(die) Slavegas(e) ist/sind - der Quotient $Q_i$ der Ist-Flüsse von Mastergas und Slavegas gebildet wird und parallel die Bildung des Quotienten $Q_S$ der Soll-Flüsse erfolgt und daß die Differenz (en) dieser Quotienten gebildet wird und das Ergebnis dieser Substraktion dem Sollfluß an Mastergas oder dem Sollfluß des Slavegases (der jeweiligen Slavegase) so hinzuaddiert wird, daß sich das Mischungsverhältnis der beteiligten Gase in Richtung des gewünschten Mischungsverhältnisses ändert.

Dieser Ablauf bildet die Basis eines schnell reagierenden, hochgenauen Regelmechanismus.

Falls ein wechselnder Mengenbedarf besteht, wird der Druck in der gemischführenden Leitung mit Vorteil in der Weise konstant eingeregelt, daß der Sollfluß an Mastergas ($F_{ms}$) (= Führungsgröße des Systems) abhängig vom Soll- und Istdruck in der gemischführenden Leitung gebildet wird,

also

$$F_{ms} = f(p_{ist}, p_{soll})$$

ist,

und- kommend von der Führungsgröße - auch die Nebengröße für den Fluß an Slavegas ($F_{ls}$) gemäß gewünschtem Mischungsverhältnis gebildet wird,

also

$$F_{ls} = g(F_{ms})$$

ist.

Durch die oben zuerst angeführte Merkmalsgruppe wird ein hinsichtlich des Mischungsverhältnisses kontrolliertes und gegebenenfalls sich nachregulierendes Gasmischverfahren angegeben, dessen Regelmechanismus auf dem Subtraktions-Vergleich zweier oder weiterer Quotienten beruht, womit besondere Vorteile, wie schnelle und empfindliche Reaktion bei nicht eingehaltenen Werten und geringe Schwingungsanfälligkeit in der technischen Realisierung mit Standard-Analogbauteilen einhergehen. Die im Falle unterschiedlicher Bedarfsmengen über den Abgabedruck ausgeführte Mengenregelung gemäß der zweiten, oben aufgeführten Merkmalsgruppe ist mit der erfindungsgemäßen Verhältnisregelung gut kombinierbar und mittels eines Festwertgebers günstig realisierbar.Die Stellgröße y des so eingesetzten Festwertgebers ist dabei gleich dem Sollfluß an Mastergas, also gleich der Führungsgröße des Regelsystems zu setzen, wobei diese Stellgröße y sich eben aus dem Ist-Abgabedruck und dem zugehörigen Solldruck ableitet.

In einer besonders vorteilhaften Ausbildung der Erfindung wird der Flußmengenregelung eine Drucksteuerung überlagert, bei der einer augenblicklich fließenden Menge eines Gases ein jeweils geeigneter Vordruck zugeordnet wird, wobei mit größeren Flußmengen größere Vordrucke eingestellt werden.

Dadurch wird erreicht, daß die bei der Realisierung des Verfahrens einzusetzenden Mengenregler jeweils in einem, in Bezug zur fließenden Menge günstigen Vordruckbereich betrieben werden. Besonders vorteilhaft ist die Realisierung jeweils günstiger Vordrucke in der Weise, daß augenblicklich fließenden Gasmengen in bestimmten Mengenbereichen jeweils eine bestimmte Vordruckstufe zugeordnet wird.

Eine Regeleinheit zur Durchführung des erfindungsgemäßen Verfahrens sowie vorteilhafte Gestaltungsvarianten dazu sind in den entsprechenden Unteransprüchen angegeben.

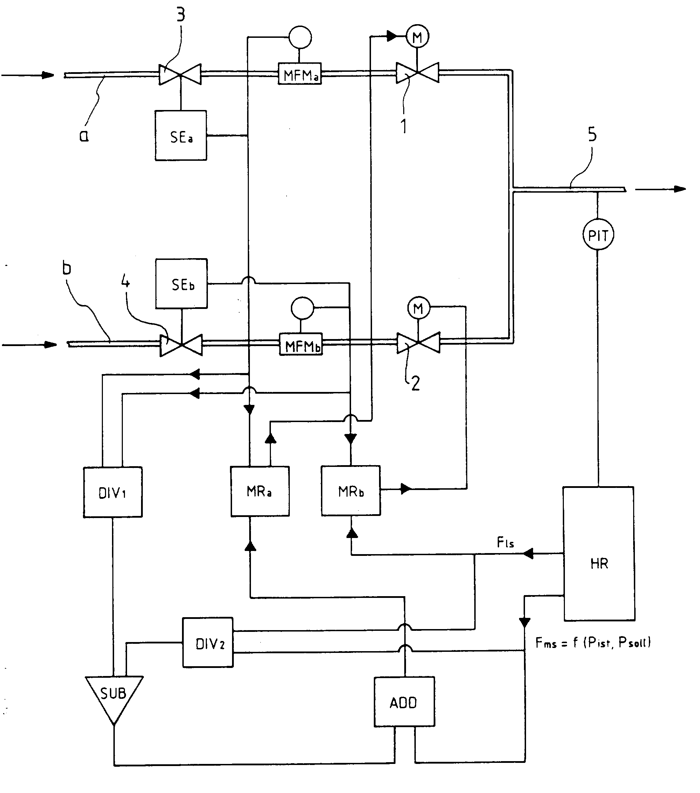

Anhand der Figur soll im folgenden die erfindungsgemäße Regelweise und eine zugehörige Regelvorrichtung beispielhaft näher erläutert werden.

Die Figur zeigt eine erfindungsgemäße Regeleinheit.

Eine erfindungsgemäße Regelvorrichtung weist einen die Hauptregelsignale bildenden Hauptregler HR auf, der mit zwei (bei mehr Mischgasen entsprechend mehr) Mengenreglern $MR_{a,b}$ verbunden ist, wobei die Verbindung zum Mengenregler für das Mastergas $MR_a$ über einen zwischengeschalteten Addierer ADD besteht, dessen zweitem Eingang die korrigierende Regelgröße für das Mastergas zugeführt ist.

Andererseits ist ein Dividierer DIV1 (oder gegebenenfalls auch mehrere) mit in den einzelnen Gaszuleitungen a, b angeordneten Mengenflußmessern MFM verbunden und bildet den Quotienten jeweils von Mastergas zu Slavegas und dieser Dividierer steht im folgenden mit einem Subtraktionsglied SUB in Verbindung, das seinerseits ausgangsseitig mit dem besagten Addierer ADD verbunden ist und diesem sein Ergebnis zuführt, wobei der zweite Eingang des Subtraktionsgliedes mit einem zweiten Dividierer DIV2 gekoppelt ist, der die Division der Sollflußwerte ausführt und dazu eingangsseitig mit den zwei Ausgängen des Hauptreglers HR in Verbindung steht.

Zudem weist eine vorteilhaft weitergebildete Regeleinheit einen Hauptregler HR auf, der mit einem Druckmesser PIT in Verbindung steht, der den Druck in der Abgabeleitung mißt, wobei aus diesem Meßwert und einem vorgegebenen Sollwert im Hauptregler HR neue Hauptregelgrößen, sprich neue Master- und Slavegasflüsse, gebildet werden.

Ferner weist eine solche Regeleinheit mit Vorteil zudem eine Verbindung der Massenflußmesser $MFM_{a,b}$ mit Steuereinheiten $SE_{a,b}$ auf, die ihrerseits mit eingangs in den Gasleitungen a, b angeordneten Druckregler 3, 4 verbunden sind.

Die Funktionsweise der Schaltung ist wie folgt:

Der Haupt- oder Verhältnisregeleinheit HR wird das Mischungsverhältnis der gewünschten, z.B. zweikomponentigen Gasmischung eingegeben, beispielsweise könnte eine Gasversorgung mit einer 3 : 1-Mischung aus Acetylen und Propylen das Ziel sein. Das gewünschte Mischungsverhältnis wird von der Hauptregeleinheit HR in ein Steuersignal $F_{ms}$ (=y) für den Soll-Fluß an Hauptkomponente - also an <u>M</u>astergas - beim zu erzeugenden Gasgemisch und in ein Steuersignal $F_{ls}$ für den Nebenkomponentenfluß (<u>S</u>lavegas) umgesetzt, wobei zunächst von einer geeigneten mittleren Gesamtbedarfsmenge ausgegangen wird.

Das $F_{ms}$-Signal wird über den Addierer ADD der Master-Mengenregeleinheit $MR_a$ zugeführt, während das $F_{ls}$-Signal direkt der Mengenregeleinheit $MR_b$ zugeleitet wird. Diese beiden Mengenregeleinheiten steuern jeweils die in den zugehörigen Gaszufuhrleitungen a und b liegenden Regelventile 1, 2 geeignet an, wodurch sich die gewünschten Gasflüsse in den Zuleitungen a und b einstellen und sich somit die gewünschte Gasversorgung eines oder mehrerer Verbraucher mit Mischgas über die gemeinsamen Hauptleitung 5 ergibt (= grundsätzlicher Funktionszustand der erfindungsgemäßen Mischgasversorgung).

Bereits dieser stationäre Funktionszustand unterliegt jedoch einer ersten Kontrolle dadurch, daß die fließenden Gasmengen mittels stromaufwärts der Regelventile 1, 2 liegenden Flußmessern $MFM_{a,b}$ gemessen werden und diese gemessenen Istwerte $F_{mi}$ und $F_{li}$ mit den vorgegebenen Sollwerten verglichen werden und gegebenenfalls eine Nachregelung der Flüsse über die zugehörigen Regelventile erfolgt, solange bis eben die jeweiligen Ist- und Sollflüsse übereinstimmen. Die von der Hauptregeleinheit vorgegebenen Sollwerte bleiben bei diesem Kontrollschritt jedoch unverändert und bis zu diesem Punkt besteht keine Kontrolle der Stimmigkeit des Verhältnisses der beiden Gasflüsse zueinander.

Diese Kontrolle wird mit der vorliegenden Schaltung im weiteren in der Weise geleistet, daß mit Hilfe des Dividierers $DIV_1$ der Quotient $Q_i = F_{mi}/F_{li}$, also der Quotient der Istwerte gebildet wird und anschließend mittels des Substraktionsgliedes SUB und dem Quotienten der Sollwerte, der durch den Dividierer $DIV_2$ hergestellt wird, ein Vergleich dieser beiden Quotienten durch Substraktion hergestellt wird.

Dabei ergibt sich - im Falle richtig eingeregelter Gasflüsse - daß $Q_i$ - $Q_s$ ist und die Substraktion somit "Null" liefert. Diese "Null" "fließt" schaltungsgemäß im weiteren dem Addierglied ADD zu und wird dort mit dem Wert $F_{ms}$ verknüpft, was im Falle der "Null" jedoch zu keiner Konsequenz führt. Ist jedoch das Ergebnis der Substraktion $Q_s$ - $Q_i$ ungleich Null, z.B. > 0, und also das Gasmischungsverhältnis nicht korrekt und im Falle > 0 der tatsächliche Fluß an Mastergas zu klein, so folgt, daß zum Basis-Sollwert $F_{ms}$ der Differenzwert im ADD-Glied hinzuaddiert wird, also

$$F'_{ms} = F_{ms} + dQ$$

gebildet wird. Dieser neue Sollwert für das Mastergas wird dann der entsprechenden Mengenregeleinheit $MR_a$ zugeführt und so eine Erhöhung des Flusses an Mastergas erzielt, während das Slavegas unverändert bleibt. Im Endeffekt wird so also eine Berichtigung des Verhältnisses der beiden Gasflüsse zueinander erreicht, was durch die erfindungsgemäße Regelung auf Quotientengleichheit regeltechnisch besonders vorteilhaft gelingt.

Der gleiche Effekt wird auch erzielt, wenn, umgekehrt wie oben, $Q_i$ - $Q_s$ gebildet wird und sich also ein negatives Subtrakionsergebnis ergibt und dies dann - wiederum über ein Addierglied - dem Sollfluß an Slavegas abgezogen wird. Damit resultiert nämlich eine Erniedrigung des Slavegasflusses während der "Master" unverändert bleibt. Insgesamt entsteht so also wieder ein in die richtige Richtung korrigiertes Mengenverhältnis der beteiligten Gase. Diese Slavegasregelung ist insbesondere bei mehr als zwei Mischgasen vorteilhaft, da damit nur die jeweils unstimmige Komponente nachgeregelt wird und die anderen Master/Slave-Mischungsverhältnisse unverändert bleiben.

Ohne weitere Maßnahmen würde der bislang beschriebene Schaltungsteil allerdings lediglich die gewünschte Gasmischung in etwa gleichbleibender Menge aber z.B. bei höherem Mengenbedarf mit sinkendem Druck liefern. Damit nun auch bei unterschiedlichen Abnahmemengen an Gas ein gleichbleibender Druck eingehalten wird, ist der oben beschriebenen Mischungsregelung vorteilhafterweise zusätzlich eine Druckregelung überlagert. Diese ist günstig in der Weise ausgeführt, daß der Druck in der Abgabeleitung 5 gemessen wird und - in Verbindung mit einem von außen vorgegebenen Solldruck - in der Hauptregeleinheit HR ein zugehöriger Sollfluß an Mastergas und von diesem kommend auch der zugehörige Sollfluß an Slavegas errechnet wird. Diese neuen Werte für Master- und Slavegas werden dann wie oben beschrieben in der Mischungsregelung erfindungsgemäß weiterverarbeitet. Auf diese Weise - nämlich über eine Druckänderung in der Abgabeleitung - werden also im gezeigten Beispiel variierende Bedarfsmengen dedektiert, wobei aus dieser Druckänderung neue Werte $F_{ms}$ und $F_{ls}$ für Master- und Slavegas(e) ermittelt werden, womit sich eine geeignete Nachregelung der Gasflüsse ergibt.

Die nunmehr weitgehend beschriebene Gasmischeinheit ist bevorzugt mit zeitlich schnell arbeitenden Bauteilen aufzubauen, um eben die Anpassung an unterschiedliche Bedarfsmengen aber auch die Reglung des richtigen Mischungsverhältnisses in befriedigendem Maß zu leisten. Dazu sind beispielsweise für die Mengenregelventile 1, 2 schrittmotorbetriebene Schieberventile von Vorteil - mit der Kombination Schrittmotor/Schieberventil läßt sich besonders schnell und genau der Strömungsquerschnitt in einer Gasleitung ändern. Außerdem sind zur Mengenmessung moderne, auf der Basis der Erwärmung von fließenden Gasen arbeitende Massendurchflußmesser besonders geeignet. Ebenso ist schnelle Rechen- und Analogtechnik einzusetzten. Sind diese Maßgaben eingehalten, so treten bei der erfindungsgemäßen Gasmischung nur geringfügige und im allgemeinen tolerierbare Druckschwankungen auf und es ist somit ein hochwertiges, ohne Pufferbehälter arbeitendes Gasmischsystem gegeben.

Eine weitere Perfektionierung der Erfindung wird schließlich dadurch erzielt, daß der bislang beschriebenen Verfahrensweise bzw. Meß- und Regelschaltung eine Vordrucksteuerung derart überlagert wird, daß - abhängig von den augenblicklich fließenden Gasmengen - bestimmte Vordrucke für die Regelventile 1, 2 über Druckregler 3, 4 eingestellt werden. Dabei werden um so größere Vordrucke eingestellt, je größer die fließenden Gasmengen sind, wodurch günstige Regelbedingungen für die Mengenregler 1, 2 hergestellt werden. Den stromaufwärts von den Mengenreglern und Massenflußmessern liegenden Druckreglern 3, 4, mit Vorteil I-P-Druckreglern, wird dabei von den zugeordneten Steuereinheiten $SE_a$, $SE_b$ die jeweils geeignete Stromstufe zugeführt und so letzlich die jeweils zugehörige Vordruckstufe erzeugt. Mit diesem stufenartig gesteuerten Vordruck werden für verschiedenste Gasmengen günstige Bedingungen für den erfindungsgemäßen Regelmechanismus hergestellt und somit die Einsetzbarkeit für unterschiedlichste Bedarfsmengen optimiert.

Insgesamt ergibt sich mit der Erfindung - insbesondere in voller Ausbaustufe - ein schnell reagierendes, hochpräzises und selbst bei geringen Gasmengen wenig schwingungsanfälliges Gasmischsystem, das insbesondere in aller Regel ohne Puffervolumen auskommt.

## Patentansprüche

1. Verfahren zur Mischung von zwei oder mehr Gasen (a,b) in geregelter Weise,

   wobei die Flüsse der unvermischten Gase geeignet im Hinblick auf die herzustellende Mischung eingestellt werden
   und wobei das Gas (a) mit dem größten Mengenanteil das Mastergas darstellt und das(die) weitere(n) Gas(e) das(die) Slavegas(e) ist/sind, **dadurch gekennzeichnet**, daß
   daß der Regelablauf in der Weise erfolgt, daß der Quotient $Q_i$ der Ist-Flüsse von Mastergas (a) und Slavegas(en) gebildet wird und parallel die Bildung des Quotienten $Q_s$ der Soll-Flüsse von Mastergas (a) und slave-gas (en) erfolgt und daß die Differenz(en) dieser Quotienten gebildet wird und das Ergebnis dieser Substraktion dem Sollfluß ($F_{ms}$) an Mastergas (a) oder dem Sollfluß ($F_{ls}$) des Slavegases (der Slavegase) so hinzuaddiert wird, daß sich das Mischungsverhältnis der beteiligten Gase in Richtung des gewünschten Mischungsverhältnisses ändert.

2. Verfahren nach Anspruch 1, **dadurch gekennzeichnet**, daß der Druck in der gemischführenden Leitung bei wechselndem Mengenbedarf in der Weise weitgehend konstant eingeregelt wird, daß der Sollfluß für das Mastergas als Führungsgröße abhängig vom Istdruck in der gemischführenden Leitung und unter Berücksichtigung eines vorgegebenen Solldrucks gebildet wird und - kommend von dieser Führungsgröße - auch die Nebengröße(n) für den Fluß (die Flüsse) an Slavegase(n) gemäß gewünschtem Mischungsverhältnis gebildet wird (werden).

3. Verfahren nach einem der Ansprüche 1 oder 2, **dadurch gekennzeichnet**, daß der Flußmengenregelung eine Drucksteuerung überlagert wird, bei der einer augenblicklich fließenden Menge ein jeweils geeigneter Vordruck zugeordnet wird, wobei mit größeren Flußmengen größere Vordrucke eingestellt werden.

4. Verfahren nach Anspruch 3, **dadurch gekennzeichnet**, daß augenblicklich fließenden Mengen in bestimmten Bereichen jeweils eine bestimmte Vordruckstufe zugeordnet wird.

5. Regeleinheit zur mischung von zwei oder mehr Gasen (a,b) zur Durchführung des Verfahrens nach einem oder mehreren der Ansprüche 1 bis 4, wobei das Gas mit dem größten Mengenanteil des Mastergas (a) darstellt und das(die) weitere(n) Gas(e) das (die) Slavegas(e) ist/sind, wobei ein die Sollflußwerte der Gase als Hauptregelsignale bildender Hauptregler (HR) mit zwei oder mehr Mengenreglern (MR$_{a,b}$) der Gaszuleitungen (a,b) verbunden ist, wobei die Verbindung zum Mengenregler für das Mastergas bzw. die zum Slavegas/zu den Slavegasen über den ersten Eingang eines zwischengeschalteten Addierers (ADD) besteht, dessen zweitem Eingang eine korrigierende Regelgröße für das Mastergas bzw. Slavegas(e) zugeführt ist,

und andererseits ein erster Dividierer (DIV$_1$) (oder auch mehrere) mit in den einzelnen Gaszuleitungen (a,b) angeordneten Mengenflußmessern (MFM a,b) verbunden ist/ sind und den Quotienten der Istflußwerte jeweils von Mastergas zu Slavegas bildet, und im folgenden eine Verbindung vom ersten Dividierer (DIV$_1$) zu einem Subtraktionsglied (SUB) besteht, das seinerseits ausgangsseitig mit dem besagten zweiten Eingang des Addierers verbunden ist und diesem sein Ergebnis zuführt,
wobei der zweite Eingang des Subtraktionsgliedes (SUB) mit einem zweiten Dividierer (DIV2) gekoppelt ist, der die Division der Sollflußwerte (F$_{ls}$, F$_{ms}$) ausführt, und dazu eingangsseitig mit den zwei Ausgängen des Hauptreglers (HR) in Verbindung steht.

6. Regeleinheit nach Anspruch 5, **dadurch gekennzeichnet**, daß der Hauptregler (HR) mit einem Druckmesser (PIT) in Verbindung steht, der den Druck in der Abgabeleitung (5) mißt, wobei aus diesem Meßwert und einem vorgegebenen Sollwert im Hauptregler (HR) neue Hauptregelgrößen gebildet werden.

7. Regeleinheit nach Anspruch 5 oder 6, **dadurch gekennzeichnet**, daß die Massenflußmesser (MFM$_{a,b}$) mit Steuereinheiten (SE$_{a,b}$) in Verbindung stehen, die ihrerseits mit eingangs in den Gaszuleitungen (a,b) angeordneten Druckreglern (3,4) verbunden sind.

**Claims**

1. Process for mixing two or more gases (a, b) in a controlled manner, wherein the flows of unmixed gases are adjusted suitably for the mixture to be produced and wherein the gas (a) present in the largest quantity represents the master gas and the further gas or gases is/are the slave gas or gases, characterised in that the control procedure is carried out in that the quotient Q$_i$ of the actual flows of master gas (a) and slave gas or gases is formed and the quotient Q$_s$ of the set flows of master gas (a) and slave gas or gases is formed in parallel and in that the difference or differences of these quotients is/are formed and the result of this subtraction is added to the set flow (F$_{ms}$) of master gas (a) or the set flow (F$_{ls}$) of the slave gas or gases so that the mixing ratio of the gases concerned alters in the direction of the desired mixing ratio.

2. Process according to claim 1, characterised in that the pressure in the line conveying the mixture is kept largely constant while the quantity requirement alters in that the set flow for the master gas is formed as the guide variable according to the actual pressure in the line conveying the mixture and allowing for a predetermined set pressure and - coming from this guide variable - the auxiliary variable or variables for the flow or flows of slave gas or gases is/are formed according to the desired mixing ratio.

3. Process according to one of claims 1 or 2, characterised in that the flow control is overlaid with a pressure control whereby a suitable pilot pressure is associated with an instantaneous flow, greater pilot pressures being set with greater flows.

4. Process according to claim 3, characterised in that a particular pilot pressure level is associated with instantaneous flows in certain ranges.

5. Control unit for mixing two or more gases (a, b) for implementing the process according to one or more of claims 1 to 4, wherein the gas present in the largest quantity represents the master gas (a) and the further gas or gases is/are the slave gas or gases, wherein a main controller (HR) forming the set flow values for the gases as main control signals is connected with two or more flow controllers (MR$_{a,b}$) of the gas supply lines (a, b), wherein the connection to the flow controller for the master gas or that for the slave gas or gases is made through the first input of an interposed adder (ADD) whose second input is fed with a correcting control variable for the master gas or slave gas or gases, and secondly a first divider (DIV$_1$) (or a plurality of dividers) is/are connected with flow meters (MFM$_{a,b}$) disposed in the individual gas supply lines (a, b) and forms the quotient of the actual flow values in each case of master gas to slave gas, and in the following a connection exists from the first divider (DIV$_1$) to a subtraction element (SUB) which in turn is connected on the output side with said second input of the adder and sends its result to the latter, wherein the second input of the subtraction element (SUB) is coupled with a second divider (DIV$_2$) which divides the set flow values (F$_{ls}$,F$_{ms}$), and for this on the input side is connected with the two outputs of the main controller (HR).

6. Control unit according to claim 5, characterised in that the main controller (HR) is connected with a manometer (PIT) which measures the pressure in the discharge line (5), new main control variables being formed from this measurement and a predetermined set value in the main controller (HR).

7. Control unit according to claim 5 or 6, characterised in that the mass flow meters (MFM$_{a,b}$) are connected with control units (SE$_{a,b}$) which in turn are connected with pressure controllers (3, 4) disposed in the gas supply lines (a, b) at the beginning.

## Revendications

1. Procédé de régulation d'un mélange de deux ou plusieurs gaz (a, b) dans lequel, les flux des gaz purs sont, avant mélange, réglés de façon convenable pour obtenir le rapport de mélange souhaité, le gaz (a) qui est le composant principal du mélange étant le gaz maître et le(s) autre(s) gaz étant le(s) gaz esclave(s), ce procédé étant caractérisé en ce que la régulation s'effectue de telle sorte que le système calcule le quotient Q$_i$ représentant le flux effectif du gaz maître (a) et de(s) gaz esclave(s) et calcule parallèlement le quotient Qs représentant les flux théoriques du gaz maître (a) et de(s) gaz esclave(s) et en ce que le système calcule la différence entre ces deux quotients, le résultat de la soustraction étant additionné au flux théorique (Fms) du gaz maître (a) ou au flux théorique (Fls) du gaz esclave (des gaz esclaves), de telle sorte que le rapport de mélange se modifie dans le sens du rapport de mélange souhaité.

2. Procédé selon la revendication 1, caractérisé en ce que la pression dans les conduites où circulent les mélanges gazeux est réglée d'une façon pratiquement constante, dans le cas de variation de la demande, de sorte que le flux théorique du gaz maître, en tant que valeur de référence, dépende de la pression effective dans la conduite où circule le mélange gazeux et est formé en tenant compte de la pression théorique prédéfinie et -à partir de cette valeur de référence- il est aussi possible de calculer la (les) valeur(s) annexe(s) du (des) flux de gaz esclave(s) selon les rapports de mélange souhaité.

3. Procédé selon la revendication 1 ou 2, caractérisé en ce qu'il comprend un système de régulation des flux, ainsi qu'un système de commande de la pression, par lequel une pression d'admission prédéfinie est attribuée à chaque quantité de gaz en circulation à un moment donné, dans la mesure où un débit plus important exige une pression d'admission plus élevée.

4. Procédé selon la revendication 2, caractérisé en ce qu'une pression d'admission déterminée est attribuée dans des domaines déterminés à chaque quantité de gaz en circulation à un moment donné.

5. Unité de régulation pour mélanger deux ou plusieurs gaz (a, b), destinée à la mise en oeuvre du procédé selon l'une ou plusieurs des revendications 1 à 4, le gaz (a) qui est le composant principal du mélange étant le gaz maître et le(s) autre(s) gaz étant le(s) gaz esclave(s), dans cette unité:

- un régulateur principal (HR), transformant les valeurs du flux théorique des gaz en signaux de régulation, étant relié à un ou plusieurs régulateurs de débit (MR$_{a,b}$) montés sur les conduites d'admission des gaz (a, b),
- le régulateur de débit du gaz maître ou du (des) flux du (des) gaz esclave(s) communiquant avec la première entrée d'un additionneur (ADD) interconnecté, dont la deuxième entrée est destinée à recevoir une valeur de régulation corrigée pour le gaz maître ou le gaz esclave,
- et, en outre, un premier diviseur (DIV1) (ou même plusieurs) étant relié aux dispositifs de mesure du débit, montés dans chaque conduite de gaz (a, b), et calculant le quotient des valeurs du flux effectif du gaz maître par rapport à chaque gaz esclave,
- et le premier diviseur (DIV1) étant relié à une unité de soustraction (SUB), dont la sortie est reliée à la deuxième entrée précitée de l'additionneur (ADD) pour transférer à ce dernier le résultat calculé,
- la deuxième entrée de l'unité de soustraction (SUB) étant couplée à un deuxième diviseur (DIV2) qui effectue la division des valeurs du flux théorique (F$_{ls}$, F$_{ms}$) et dont l'entrée communique avec les deux sorties du régulateur principal (HR).

6. Unité de régulation selon la revendication 5, caractérisée en ce que le régulateur principal (HR) est relié à un indicateur de pression (PIT), qui mesure la pression dans la conduite de sortie (5), de nouvelles valeurs de régulation étant calculées dans le régulateur principal (HR) à partir de la valeur de mesure de la pression et d'une valeur théorique prédéfinie.

7. Unité de régulation selon la revendication 5 ou 6, caractérisée en ce que le dispositif de mesure du débit ($MFM_{a,b}$) est relié à des unités de commande ($SE_{a,b}$), dont l'entrée de chacune est reliée à des régulateurs de pression (3, 4) montés dans les conduites d'admission des gaz (a, b).

$F_{ls}$

$F_{ms} = f (P_{ist}, P_{soll})$